# EUROPEAN PATENT APPLICATION

(11) **EP 4 450 973 A1**
(43) Date of publication of application: **23.10.2024**
(21) Application number: 22906668.3
(22) Date of filing: 15.12.2022
(51) Int. Cl.: G01N 33/68, C07K 14/525

(54) **NEW TARGETS FOR DIAGNOSING AND TREATING ALZHEIMER'S DISEASE AND USE THEREOF**

(30) Priority: 15.12.2021 CN 202111539714
(71) Applicant: Shanghai JW Inflinhix Co., Ltd., Shanghai 201203 (CN)
(72) Inventor: JIANG, Shisong, Shanghai 200135 (CN); LU, Wenshu, Shanghai 200135 (CN); ZHU, Renying, Shanghai 200135 (CN); JIAN, Aili, Shanghai 200135 (CN)
(74) Representative: Impact Intellectual Property LLP
(86) International application number: PCT/CN2022/139407
(87) International publication number: WO 2023/109923

(57) **Abstract**

The present invention relates to targets for diagnosing and treating Alzheimer's disease. The present invention further provides a method for diagnosing and treating Alzheimer's disease by means of the targets. The diagnosis of Alzheimer's disease by means of the method of the present invention has the advantages of simple material taking, a low price, rapid detection and objective and accurate detection results, etc.

## Description

### Technical field

The present invention relates to the field of medicine. In particular, the present invention relates to new targets for the diagnosis and treatment of dementia, and applications of these targets for the diagnosis and treatment of dementia.

### Background

dementia is a neurodegenerative disease with a decline in the advanced cognitive function as the main clinical manifestation, characterized by progressive mental decline, cognitive dysfunction and personality changes. With the development of population aging, the incidence of dementia is dramatically increased year by year, bringing heavy economic and mental burden to patients and their families, and dementia has become a public social problem that needs to be solved globally. Dementia includes various types, including Alzheimer's disease (AD) and vascular dementia (VD) as the most common types, accounting for more than 90% of all patients with dementia. AD is a progressive neurodegenerative disease with clinical manifestations of cognitive dysfunction and memory loss, the pathology of which is characterized by diffuse cortical atrophy, age spots caused by abnormal extracellular deposition of amyloid beta (Aβ), and neuronal fibrillary tangles (NFTs) caused by aggregates of Tau protein in nerve cells. With the aging of the population, it is expected that by 2050, 100 million people worldwide will suffer from AD. VD is the second most common dementia disease after AD, accounting for about 15% of dementia diseases. Acute local ischemia caused by cerebral reperfusion, long-term chronic hypoperfusion, long-term hypertensive state, hyperglycemia, aging and hydrocephalus can induce blood-brain barrier function impairment, resulting in changes in the cerebrovascular structure that ultimately lead to VD. Blood-brain barrier function impairment plays an important role in the pathomechanism of VD.

Currently, main tests for dementia are limited to neuroimaging and neuropsychological tests (in the form of scales). Since the cause and mechanism of dementia have not yet been elucidated, there are no specific drugs for treating dementia, nor are there specific biochemical and immunological diagnostic methods. Scholars believe that it is mainly related to the death of nerve cells caused by various factors such as genes and environment. Most studies have focused on the development of diagnostic and therapeutic approaches using Aβ and Tau as biological targets. Currently, most clinical trials failed. Although a treatment targeting Aβ was recently approved by the US FDA, it was reported that the approval process was markedly controversial.

In summary, no effective diagnosis and treatment methods are developed in the current research. The "holy grail" in this field will be the discovery of biological targets that are more relevant to the causes of dementia and the development of diagnostic and therapeutic programs around this or these biological targets.

### Summary of the Invention

The purpose of the present invention is to provide novel diagnostic and therapeutic targets for dementia.

In a first aspect, the present invention provides a use of a TNF-derived polypeptide, wherein said TNF-derived polypeptide is used: (1) as a marker for the diagnosis or detection of dementia; (2) in the preparation of a reagent or kit for the diagnosis or detection of dementia; or (3) in the preparation of a medicament for treating dementia.

In a specific embodiment, said TNF-derived polypeptide is a polypeptide selected from one or more of the following:
1) a polypeptide having an amino acid sequence shown in any one of SEQ ID NO: 2-26; or,
2) a polypeptide having a sequence similarity of at least 80% or higher, 85% or higher, 90% or higher, 95% or higher, 96% or higher, 97% or higher, 98% or higher, 99% or higher, to a polypeptide having an amino acid sequence shown in any one of SEQ ID NO: 2-26.

In a specific embodiment, said dementia is Alzheimer's disease or vascular dementia.

In a second aspect, the present invention provides a kit for diagnosing or detecting dementia, wherein said kit comprises a detection reagent and a container holding said detection reagent,
said detection reagent detects the presence of a TNF-derived polypeptide or an antibody capable of binding to said TNF-derived polypeptide in the serum of a subject to be tested, and said TNF-derived polypeptide is:
1) a polypeptide having an amino acid sequence shown in any one of SEQ ID NO: 2-26; or
2) a polypeptide having a sequence similarity of at least 80% or higher, 85% or higher, 90% or higher, 95% or higher, 96% or higher, 97% or higher, 98% or higher, 99% or higher, to a polypeptide having an amino acid sequence shown in any one of SEQ ID NO: 2-26.

In a preferred embodiment, said kit further comprises an instruction for use that teaches an operator on how to diagnose or detect dementia using the detection reagent or kit.

In a third aspect, the present invention provides a TNF-derived polypeptide, wherein said TNF-derived polypeptide is
1) a polypeptide having an amino acid sequence shown in any one of SEQ ID NO: 2-26; or
2) a polypeptide having a sequence similarity of at least 80% or higher, 85% or higher, 90% or higher, 95% or higher, 96% or higher, 97% or higher, 98% or higher, 99% or higher, to a polypeptide having an amino acid sequence shown in any one of SEQ ID NO: 2-26.

In a preferred embodiment, said TNF-derived polypeptide is used: (1) as a marker for the diagnosis or detection of dementia; (2) in the preparation of a reagent or kit for the diagnosis or detection of dementia; or (3) in the preparation of a medicament for the treatment of dementia.

In a preferred embodiment, said dementia is Alzheimer's disease or vascular dementia.

In a fourth aspect, the present invention provides a combination of two or more TNF-derived polypeptides, wherein said TNF-derived polypeptide is:
1) a polypeptide having an amino acid sequence shown in any one of SEQ ID NO: 2-26; or
2) a polypeptide having a sequence similarity of at least 80% or higher, 85% or higher, 90% or higher, 95% or higher, 96% or higher, 97% or higher, 98% or higher, 99% or higher, to a polypeptide having an amino acid sequence shown in any one of SEQ ID NO: 2-26.

In a preferred embodiment, said combination of TNF-derived polypeptide is used: (1) as a marker for the diagnosis or detection of dementia; (2) in the preparation of a reagent or kit for the diagnosis or detection of dementia; or (3) in the preparation of a medicament for the treatment of dementia.

In a preferred embodiment, said dementia is Alzheimer's disease or vascular dementia.

In a fifth aspect, the present invention provides a binding agent or antagonist of TNF-derived polypeptide, wherein said TNF-derived polypeptide is:
1) a polypeptide having an amino acid sequence shown in any one of SEQ ID NO: 2-26; or
2) a polypeptide having a sequence similarity of at least 80% or higher, 85% or higher, 90% or higher, 95% or higher, 96% or higher, 97% or higher, 98% or higher, 99% or higher, to a polypeptide having an amino acid sequence shown in any one of SEQ ID NO: 2-26.

In a preferred embodiment, said binding agent or antagonist includes, but is not limited to, an antibody, a small molecule compound that binds or antagonizes said TNF-derived polypeptide or, a protein or peptide that binds or antagonizes said TNF-derived polypeptide.

In a preferred embodiment, said dementia is Alzheimer's disease or vascular dementia.

In a sixth aspect, the present invention provides a use of a binding agent or antagonist of a TNF-derived polypeptide for the preparation of a medicament for treating dementia, wherein said TNF-derived polypeptide is:
1) a polypeptide having an amino acid sequence shown in any one of SEQ ID NO: 2-26; or
2) a polypeptide having a sequence similarity of at least 80% or higher, 85% or higher, 90% or higher, 95% or higher, 96% or higher, 97% or higher, 98% or higher, 99% or higher, to a polypeptide having an amino acid sequence shown in any one of SEQ ID NO: 2-26.

In a preferred embodiment, said binding agent or antagonist includes, but is not limited to, an antibody, a small molecule compound that binds or antagonizes said TNF-derived polypeptide or, a protein or peptide that binds or antagonizes said TNF-derived polypeptide.

In a preferred embodiment, said dementia is Alzheimer's disease or vascular dementia.

In a seventh aspect, the present invention provides a pharmaceutical composition comprising a binding agent or antagonist of a TNF-derived polypeptide and optionally a pharmaceutically acceptable excipient, wherein said TNF-derived polypeptide is:
1) a polypeptide having an amino acid sequence shown in any one of SEQ ID NO: 2-26; or
2) a polypeptide having a sequence similarity of at least 80% or higher, 85% or higher, 90% or higher, 95% or higher, 96% or higher, 97% or higher, 98% or higher, 99% or higher, to a polypeptide having an amino acid sequence shown in any one of SEQ ID NO: 2-26.

In a preferred embodiment, said binding agent or antagonist includes, but is not limited to, an antibody, a small molecule compound that binds or antagonizes said TNF-derived polypeptide or, a protein or peptide that binds or antagonizes said TNF-derived polypeptide.

In a preferred embodiment, said dementia is Alzheimer's disease or vascular dementia.

In an eighth aspect, the present invention provides a method for detecting or diagnosing dementia, said method comprising:
1) detecting the presence of a TNF-derived polypeptide or an antibody capable of binding to said TNF-derived polypeptide in the serum of a subject to be tested, wherein said TNF-derived polypeptide is a polypeptide having an amino acid sequence shown in any one of SEQ ID NO: 2-26, or a polypeptide having a sequence similarity of at least 80% or higher, 85% or higher, 90% or higher, 95% or higher, 96% or higher, 97% or higher, 98% or higher, 99% or higher, to a polypeptide having an amino acid sequence shown in any one of SEQ ID NO: 2-26;
2) determining that the subject to be tested has dementia, if said TNF-derived polypeptide or an antibody capable of binding to said TNF-derived polypeptide is present in the serum of the subject to be tested.

In a preferred embodiment, said dementia is Alzheimer's disease or vascular dementia.

In a ninth aspect, the present invention provides a prognostic method for dementia, said method comprising:
1) detecting the presence of a TNF-derived polypeptide or an antibody capable of binding to said TNF-derived polypeptide in the serum of a subject to be tested, wherein said TNF-derived polypeptide is a polypeptide having an amino acid sequence shown in any one of SEQ ID NO: 2-26, or a polypeptide having a sequence similarity of at least 80% or higher, 85% or higher, 90% or higher, 95% or higher, 96% or higher, 97% or higher, 98% or higher, 99% or higher, to a polypeptide having an amino acid sequence shown in any one of SEQ ID NO: 2-26;
2) determining that the subject to be tested having a poor prognosis for dementia, if said TNF-derived peptide or an antibody capable of binding to said TNF-derived peptide is present in the serum of a subject to be tested.

In a preferred embodiment, said dementia is Alzheimer's disease or vascular dementia.

It should be understood that, each of the above-described technical features of the present invention and each of the technical features specifically described below (e.g., in the embodiments) may be combined with each other within the scope of the present invention, thereby constituting a new or preferred technical solution, which will not be described herein one by one due to the limited contents of the specification.

### Description of drawings

Figure 1 shows a comparison of TNFα antibodies, TNF-Ab, in the serum from patients with Alzheimer's disease, vascular dementia, the control group of the same age, and healthy individuals;
Figure 2 shows a comparison ofP1516-Ab in the serum from patients with Alzheimer's disease and the control group of the same age;
Figure 3 shows a comparison ofP1516-Ab in the serum from patients with Alzheimer's disease and 100 healthy individuals;
Figure 4 shows a comparison ofP1516-Ab concentrations in the serum from patients with vascular dementia and the control group of the same age;
Figure 5 shows a comparison ofP1516-Ab concentrations in the serum from patients with vascular dementia and 100 healthy individuals;
Figure 6 shows a comparison ofP141516-Ab concentrations in the serum from patients with VD and the control group of the same age;
Figure 7 shows a comparison of P141516 T-Ab concentrations in the serum from patients with VD and the control group of the same age;
Figure 8 shows a comparison *of in vivo* P1213-Ab concentrations in patients and the control group of the same age;
Figure 9 shows a comparison *of in vivo* P1213-Ab concentrations in patients and 100 healthy individuals;
Figure 10 shows a comparison *of in vivo* P1920-Ab concentrations in patients and the control group of the same age;
Figure 11 shows a comparison *of in vivo* P1920-Ab concentrations in patients and 100 healthy individuals;
Figure 12 shows the major sequences targeted by P1516 antibodies in patients and results of serum controls of healthy individuals;
Figure 13 shows sequences which are TNF-α sequences, and the different colored fragments represent fragments of different amino acid sizes from the sera of dementia patients detected in mass spectrometry assays. The locations of P1213, P1516, and P1920 are indicated by blue arrows. Most of the fragments detected in the serum of the patient are concentrated in these regions;
Figure 14. HMC3 cells (1 × 10⁴/well) were co-cultured with LPS (1 ug/ml), IL-4 (20 ng/ml), TNF (20 ng/ml), and a series of TNF-derived polypeptides (50 uM/ml, P78-P2122), respectively, for 24 h, and 3 replicate wells were prepared for each group; afterwards, cck8 reagent was added and co-cultured with cells for another 1h; and then, the absorbance at OD450ₙₘ was detected. C1: cell only, C2: LPS, C3: IL-4;
Figure 15 compares the use of the polypeptides of the present invention alone or in combination for detecting relevant antibodies in sera from Alzheimer's disease (AD), vascular dementia (VD), the control group of the same age (Control), and healthy individual controls (Health).

### Modes for carrying out the invention

Upon extensive and in-depth research, the inventors unexpectedly discovered a series of TNF-derived polypeptides which have a clear correlation with dementia, and thus can be used for diagnosing or treating dementia, based on which the present invention has been completed.

### TNF and TNF-derived polypeptides

Tumor necrosis factor (TNF) is a multifunctional cytokine that can promote cell proliferation, cause inflammatory reactions, and kill tumor cells. It is secreted by various cells, but mainly macrophages and T cells, in which monocyte macrophages secrete TNF-α, commonly known as TNF; activated T cells secrete lymphotoxins, also known as TNF-β. TNF is the cytokine, the level of which is firstly elevated in the inflammatory response, and named after its ability to induce rapid haemorrhagic necrosis of cancers in experiment is discovered. It has profound and diverse effects on the physiology and pathology of the body and is closely associated with a wide range of acute and chronic inflammatory diseases.

Tumor necrosis factor (tumor necrosis factor-α, TNF-α) is associated with many inflammatory diseases. However, research on TNF in dementia is not consistent. It was found in some studies that the proportion of dementia in patients with inflammatory diseases related to tumor necrosis factor is higher than that in the control group. However, neutralizing antibodies that can block the action of TNF have little effect on the treatment of dementia. There was no significant correlation between TNF and dementia in the peripheral blood.

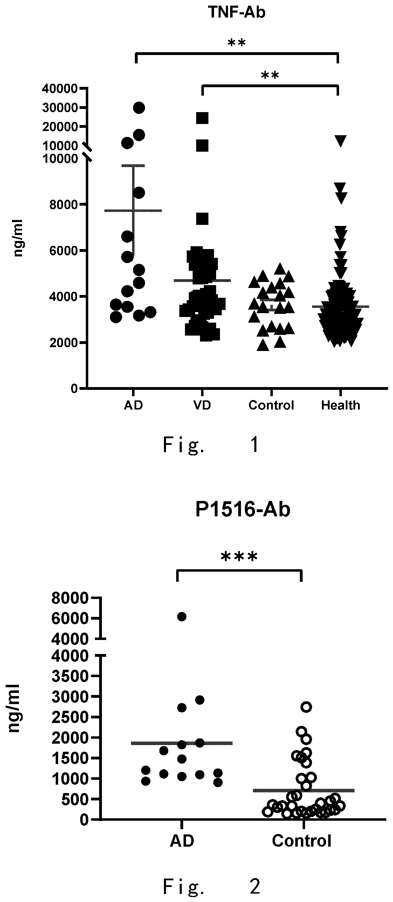

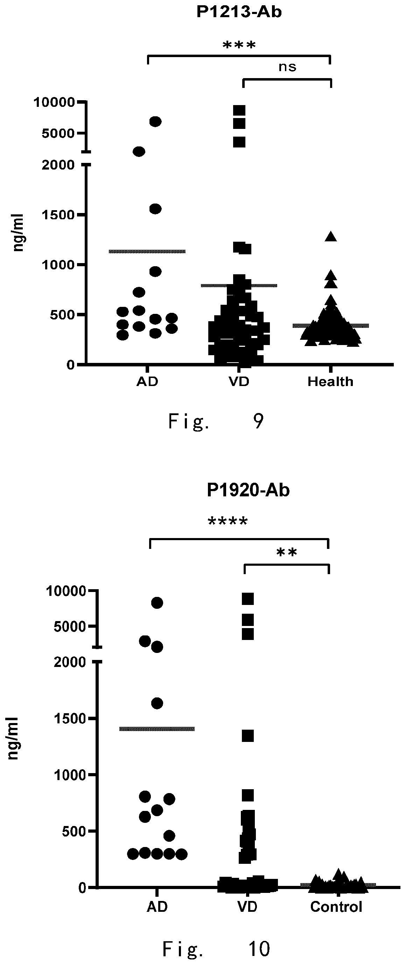

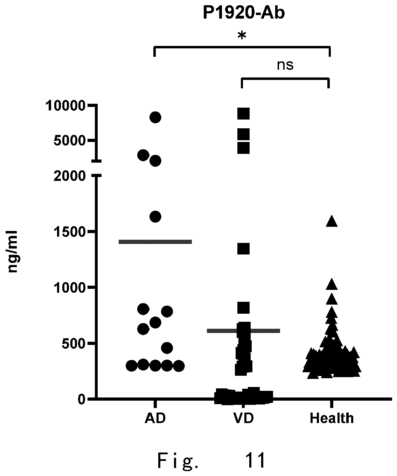

During the research process, the inventors unexpectedly found that there was no significant correlation between peripheral blood TNF levels and dementia, however there is a significant correlation between TNF antibody levels, as well as TNF derived peptides P1516, P1213, P1920, and a series of polypeptides of 10-20 amino acids derived from P1516, and dementia (Figure 1-12). Antibodies targeting TNF or TNF derived polypeptides could be detected in the serum of dementia patients as being significantly higher than those in healthy individuals or other disease patients.

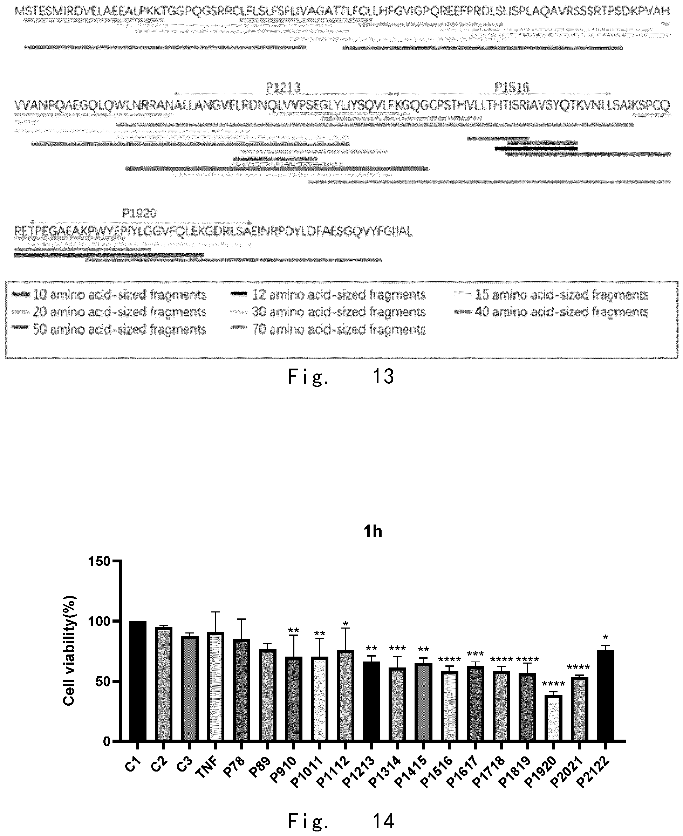

Furthermore, the inventors confirmed that, through mass spectrometry detection results, there are indeed polypeptide fragments, which have similar size and relatively high sequence similarity to P1213, P1516, P1920, and the like, in the serum of dementia patients with high levels of antibodies against the derived polypeptide (Figure 13).

Therefore, the TNF derived polypeptide of the present invention can be a polypeptide having an amino acid sequence shown in any one of SEQ ID NOs: 2-26, or the TNF derived polypeptide of the present invention can be a polypeptide with a sequence similarity of at least 80%, 85%, 90%, 95%, 96%, 97%, 98%, and 99% with an amino acid sequence shown in anu one of SEQ ID NOs: 2-26.

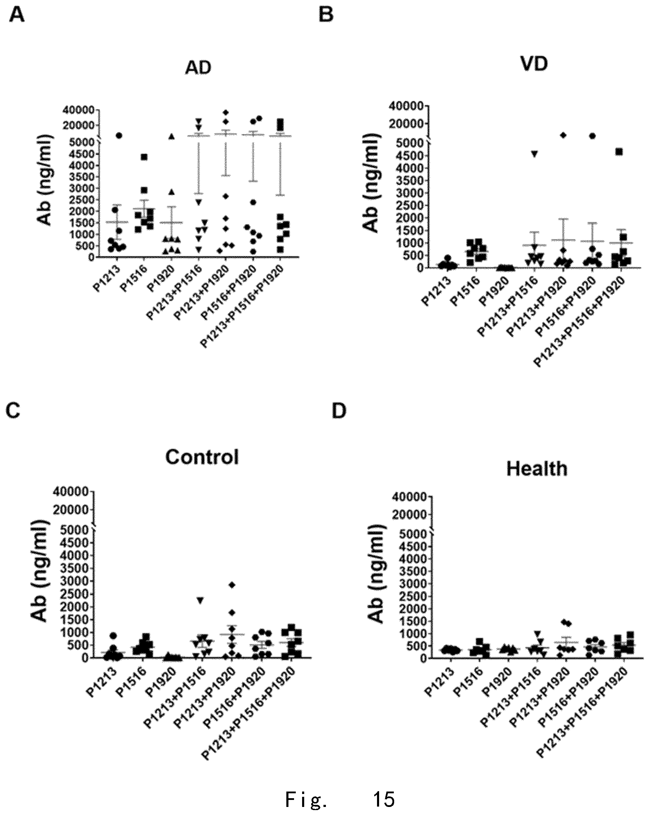

The present inventor further discovered that, when the polypeptides of the present invention is used alone or in combination to detect relevant antibodies in the serum of patients of Alzheimer's disease (AD), vascular dementia (VD), and controls of the same age (Control) and healthy individuals (Health), the sensitivity of the combined use to detect relevant autoantibodies is higher (Figure 15). Therefore, the TNF derived polypeptides of the present invention can also be a combination of two or more of the above-mentioned polypeptides.

### Use of TNF derived polypeptides of the present invention

Through *in vitro* cell experiments, the inventors have confirmed that the TNF derived polypeptides can effectively kill human neural tissue microglial cells HMC3 (Figure 14). Therefore, these polypeptides can be used as biological targets for the diagnosis and treatment of dementia. Detecting antibodies against these polypeptides and regulating these biological targets can help the early intervention for dementia patients and prevent the occurrence and development of dementia. The series of polypeptides of the present invention may be used in the clinical diagnosis of AD and VD, and corresponding treatment plans can be developed based on these polypeptides.

Based on the above findings, the present invention provides the use of TNF derived polypeptides, wherein the TNF derived polypeptides can be: (1) used as biomarkers for diagnosing or detecting dementia; (2) used in the preparation of a reagent or kit for the diagnosis or detection of dementia; or (3) used in the preparation of a medicament for treating dementia.

### Kits

The present invention also provides a kit for the diagnosis or detection of dementia, wherein the kit comprises a detection reagent and a container containing the detection reagent. The detection reagent detects the presence of TNF derived polypeptides or antibodies capable of binding to the TNF derived polypeptides in the serum of a subject to be tested. The kit may also include an instruction teaching an operator on how to use the detection reagent to diagnose or detect dementia.

Based on the above-mentioned TNF derived polypeptides or kit, the present invention also provides a method for the detection, diagnosis, or prognosis of dementia. The method includes detecting the presence of TNF derived polypeptides or antibodies capable of binding to the TNF derived polypeptides in the serum of a subject to be tested; and, if the TNF derived polypeptides or antibodies capable of binding to the TNF derived polypeptides are present in the serum of the subject to be tested, it is determined that the subject to be tested has dementia or a poor prognosis.

### Antagonists of TNF derived polypeptides of the present invention

As used herein, the term "antagonist" has a meaning as commonly understood by a skilled person, which refers to a substance that, after binding to a receptor or receptor like substance, does not cause biological effects but blocks the action mediated by the receptor agonist.

The present inventors have demonstrated that said TNF-derived polypeptide can effectively killing human neural tissue microglia HMC3. Therefore, antagonists of said TNF-derived polypeptide can be prepared into a medicament for treating dementia.

In a specific embodiment, antagonists of the TNF-derived polypeptide of the present invention include, but are not limited to, antibodies, small molecule compounds having antagonistic effects on the TNF-derived polypeptide of the present invention, proteins or polypeptides having antagonistic effects on the TNF-derived polypeptide of the present invention. In a preferred embodiment, the antagonist of the TNF-derived polypeptide of the invention may be an inhibitor of said TNF-derived polypeptide.

Based on the antagonist of the TNF-derived polypeptide, the present invention also provides a pharmaceutical composition comprising said antagonist. The pharmaceutical compositions may be used for treating dementia, such as Alzheimer's disease or vascular dementia.

### Main advantages of the present invention:

1. simple sampling: the polypeptide of the present invention can be used for the diagnosis of dementia, only a small amount of peripheral blood can be extracted from a patient for testing, and it is not necessary to extract the cerebrospinal fluid, which will reduce the pain of the patient;
2. inexpensive price: compared with the PET, CT and MRI examination performed on the head, the price is inexpensive;
3. rapid detection: results are available in as little as 2 hours;
4. results of the test method of the present invention are read by a machine and is relatively objective and accurate as compared with the scale, which is subjective.

Technical solutions of the present invention will be further described below in connection with specific embodiments, however following embodiments do not constitute a limitation of the present invention, and all the various embodiments based on the principles and technical means of the present invention shall fall within the scope of the present invention. Experimental methods for which specific conditions are not indicated in the following embodiments are generally in accordance with conventional conditions, or in accordance with conditions recommended by manufacturers. Percentages and portions are by weight unless otherwise indicated.

### Example 1. Synthesis of polypeptides

P1516, P1213 and P1920 are polypeptides of 30 amino acid in full length, which were synthesized by Ontolax (Hangzhou, China).

Based on the full length of P1516, polypeptides of 10 amino acids and 20 amino acids in length were designed and synthesized by Special Peptide Company (Hangzhou, China), respectively. Among them, polypeptides 10-1 to 10-21 were 10 amino acids in length, and each of them had 9 amino acids overlapping with the previous one except for 10-1. Polypeptides 20-1 to 20-11 were 20 amino acids in full length, and each of them had 19 amino acids overlapping with the previous one except for 20-1.

P141516 is a polypeptide of 40 amino acids in full length. P141516-Trun is a polypeptide synthesized by knocking out 5 amino acid residues at the head end and 4 amino acid residues at the tail end of P141516. The sequences of all the synthesized polypeptides are shown in the table below:

| | |
|---|---|
| P141516: LYLIYSQVLFKGQGCPSTHVLLTHTISRIAVSYQTKVNLL (SEQ ID NO: 1) | |
| P1516: KGQGCPSTHVLLTHTISRIAVSYQTKVNLL (SEQ ID NO: 2) | |
| P1213: ALLANGVELRDNQLVVPSEGLYLIYSQVLF (SEQ ID NO: 3) | |
| P141516-trun: SQVLFKGQGCPSTHVLLTHTISRIAVSYQTK (SEQ ID NO: 4) | |
| P1920: TPEGAEAKPWYEPIYLGGVFQLEKGDRLSA (SEQ ID NO: 5) | |
| 10-1: KGQGCPSTHV (SEQ ID NO: 6) | 20-1: KGQGCPSTHVLLTHTISRIA (SEQ ID NO: 27) |
| 10-2: GQGCPSTHVL (SEQ ID NO: 7) | 20-2: GQGCPSTHVLLTHTISRIAV (SEQ ID NO: 28) |
| 10-3: QGCPSTHVLL (SEQ ID NO: 8) | 20-3: QGCPSTHVLLTHTISRIAVS (SEQ ID NO: 29) |
| 10-4: GCPSTHVLLT (SEQ ID NO: 9) | 20-4: GCPSTHVLLTHTISRIAVSY (SEQ ID NO: 30) |
| 10-5: CPSTHVLLTH (SEQ ID NO: 10) | 20-5: CPSTHVLLTHTISRIAVSYQ (SEQ ID NO: 31) |
| 10-6: PSTHVLLTHT (SEQ ID NO: 11) | 20-6: PSTHVLLTHTISRIAVSYQT (SEQ ID NO: 32) |
| 10-7: STHVLLTHTI (SEQ ID NO: 12) | 20-7: STHVLLTHTISRIAVSYQTK (SEQ ID NO: 33) |
| 10-8: THVLLTHTIS (SEQ ID NO: 13) | 20-8: THVLLTHTISRIAVSYQTKV (SEQ ID NO: 34) |
| 10-9: HVLLTHTISR (SEQ ID NO: 14) | 20-9: HVLLTHTISRIAVSYQTKVN (SEQ ID NO: 35) |
| 10-10: VLLTHTISRI (SEQ ID NO: 15) | 20-10: VLLTHTISRIAVSYQTKVNL (SEQ ID NO: 36) |
| 10-11: LLTHTISRIA (SEQ ID NO: 16) | 20-11: LLTHTISRIAVSYQTKVNLL (SEQ ID NO: 37) |
| 10-12: LTHTISRIAV (SEQ ID NO: 17) | |
| 10-13: THTISRIAVS (SEQ ID NO: 18) | |
| 10-14: HTISRIAVSY (SEQ ID NO: 19) | |
| 10-15: TISRIAVSYQ (SEQ ID NO: 20) | |
| 10-16: ISRIAVSYQT (SEQ ID NO: 21) | |
| 10-17: SRIAVSYQTK (SEQ ID NO: 22) | |
| 10-18: RIAVSYQTKV (SEQ ID NO: 23) | |
| 10-19: IAVSYQTKVN (SEQ ID NO: 24) | |
| 10-20: AVSYQTKVNL (SEQ ID NO: 25) | |
| 10-21: VSYQTKVNLL (SEQ ID NO: 26) | |

### Example 2. Testing of serum from Patients

### I. Admission criteria for serum specimens from patient

### Admission criteria for AD:

1. fulfiling diagnostic criteria for organic mental disorders;
2. comprehensive intellectual impairment;
3. no sudden stroke-like episodes and no signs of focal neurological damage in the early stages of the disease;
4. no clinical or special examination suggesting that the intellectual impairment is due to other somatic or brain disorders;
5. Following features may support the diagnosis but are not essential:
   5.1 Impairment of higher cortical function, which may include aphasia, dysarthria and dyspraxia;
   5.2 Apathy, lack of active activity, or irritability and loss of control of social behaviour;
   5.3 Parkinsonian symptoms and seizures may be present in advanced severe cases;
   5.4 Somatic, neurological system, and may have laboratory tests demonstrating brain atrophy;
   6 Neuropathological examination helps to confirm the diagnosis.

### Exclusion criteria for AD:

Intellectual impairment due to other organic brain lesions such as cerebrovascular disease, pseudodementia due to mental disorders such as depression, mental retardation, or benign amnesia in the elderly were excluded.

### Admission criteria for VD:

1. Male or female outpatients or inpatients aged 50-85 years;
2. Primary school education or above;
3. Patients with cranial MRI showing cerebral white matter high signal of vascular origin (Fazekas score & 1) and & 1 lacunar lesion. 4. patients with at least one of the following clinical manifestations;
4. Meeting at least one of the following clinical manifestations:
   ① Previous clinical history of lacunar stroke syndrome (acute lacunar infarction syndrome with symptomatic associated new lesions ≤ 20 mm in the subcortical white matter or basal ganglia on cranial MRI);
   ② Asymptomatic or cognitive dysfunction (abnormalities in memory and/or other cognitive domains persisting for at least 3 months), which is likely to be causally related to cerebrovascular disease and manifests itself as a sudden or fluctuating, step-like progression of cognitive decline; cognitive decline may be a decline in executive functioning, inattentiveness, subcortical-type mild cognitive impairment, subcortical vascular dementia;
   ③ Emotional-affective abnormalities: depressive states or anxiety states;
   ④ Bladder dysfunction: urinary incontinence, etc;
   ⑤ Gait abnormalities: Parkinsonian-like gait, etc;
   ⑥ Pseudobulbar palsy: dysphagia, dysarthria, strong crying and laughing, etc;
5. Sign the informed consent form.

### Exclusion criteria for VD:

1. Patients with new TOAST-stratified non-lacunar cerebral infarction or intracranial haemorrhagic disease within 6 months;
2. Comorbid hydrocephalus and other cerebral white matter lesions of non-vascular origin (e.g., multiple sclerosis, carbon monoxide toxic encephalopathy, etc.);
3. Systemic diseases that may lead to cognitive impairment (e.g., hepatic and renal insufficiency, endocrine disorders, vitamin deficiencies, systemic autoimmune diseases, etc.);
4. Associated depression (Hamilton Depression Scale score & 17) or other unrelated serious mental illness (schizophrenia, bipolar disorder, or delirium); and
5. Severe neurological disorders such as CNS infections, Creutzfeldt-Jakob disease, Huntington's chorea and primary Parkinson's disease, dementia with Lewy bodies, corticobasal ganglia degeneration, traumatic brain injury, epilepsy, brain tumors;
6. Patients with a combination of severe cardiac, pulmonary and renal insufficiency (creatinine > 2.0 mg/dl or 177 µmοl/L), severe hepatic impairment (aminotransferases more than 3 times the normal value), malignant tumours, etc., with a life expectancy of less than 2 years;
7. Patients with coagulation disorders or thrombocytopenia (platelets < 100 × 10⁹/L);
8. Patients who cannot undergo MRI;
9. Other cerebral small-vessel diseases, including (1) hereditary small-vessel disease; (2) inflammatory or immune-mediated small-vessel disease; (3) venous collagenisation disease, etc;
10. Patients who are pregnant, breastfeeding or have the possibility of pregnancy, and those who plan to become pregnant;
11. Patients who have participated in other interventional clinical studies within the last 3 months or are participating in other interventional clinical studies
12. Illiteracy or the presence of uncorrectable visual or hearing impairment that prevents the completion of neuropsychological tests and scales;

### Requirements on Control group:

1. Persons aged 60-80 years who are medically healthy;
2. absence of autoimmune diseases;
3. absence of vascular disease;
4. absence of infarct lesions;
5. no sudden stroke-like episodes and no signs of focal neurological damage in the early stages of the disease;
6. no history of prolonged dizziness or posterior circulation ischaemia.

### III. ELISA assay

### Indirect Elisa was used in this experiment.

1. Day 1: 100 ul/well of coating solution (5 ug/ml of polypeptide), seal the membrane in a 4°C refrigerator overnight;
2. Day 2:
   ① washing solution at 200 ul/well for 5 minutes, washing the plate once;
   ② blocking solution at 100 ul/well, sealing the membrane in an electric thermostat incubator for 2 hours;
   ③ Taking the plate after 2 hours, adding the sample and standard at 100 ul/well, sealing the membrane in the electric constant temperature incubator for 1 hour;
   ④ Taking the plate after 1 hour, washing solution at 200 ul/well for 5 times;
   ⑤ HRP-labelled goat anti-human antibody at 50 ul/well, sealing the membrane and placing the plate at room temperature for 30 minutes;
   ⑥ Repeating step ④;
   ⑦ Color development solution at 100 ul/well in darkness and developing for 5-10 minutes;
   ⑧ Quenching solution at 50 ul/well, reading the OD value at OD450nm.

### IV. Experiment on glial cell

1. Day 1: HMC3 cells were plated at 1*10^4 cells/well and left to attach to the wall;
2. Day 2: diluted drugs, LPS (1 ug/ml), IL-4 (20 ng/ml), TNF (20 ng/ml), TNF-related peptide (50 uM/ml) were added according to the layout in triplicate for each group, and co-cultured for 24h;
3. Day 3: after 24h, the plate was taken, cck8 reagent was added at 10 ul/well, and then co-cultured with cells for 1 h to detect the absorbance at OD450nm.

### V. Detection through mass spectrometry

### 1. Sample Preparation:

1.1 Experimental materials:
   A. patient serum:
   B. filler coupled with protein G
1.2 Experimental methods and steps:
   A. coupling Protein G to serum with a high content of autoantibodies against TNF polypeptide:
      ① 250 ul of protein G filler was taken, and 500 ul of pure water was added, centrifuged at 1000 rpm for 2 minutes, washed for 2 times, and then washed with PBS for 1 time.
      ② 30 ul of serum from a patient was taken (1: 25 dilution), and 50 ul of serum after dilution was taken and used as a sample before adsorption for the concentration test at a later stage.
      ③ The remaining diluted serum was co-incubated with Protein G filler for 1 h. After incubation, 50 ul of serum was taken and used as a post-adsorption sample for concentration test at a later stage.
      ④ The rest was centrifuged at 1500 rpm for 5 min, and the supernatant was removed.
      ⑤ The Protein G filler with adsorbed antibodies in the serum was used for the next adsorption experiment.
   B. Protein G with adsorbed antibodies was co-incubated with serum samples from the dementia patient group with a high TNF concentration, and eluted with 500 ul of 0.1 M pH 3.0 Glyine, and the eluted material was sent for mass spectrometry. Chemically synthesised P1516 (50 mg/ml) was diluted at 50-fold in PBS to 1 mg/ml and used as a control for the above test and sent for mass spectrometry.

### 2. Detectin of Molecular weight through mass spectrometry

2 µl of PNGas F was added into an appropriate amount of sample at 37°C for 2 h and then uploaded for mass spectrometry analysis to detect the complete molecular weight of the sample.

### 3. Full sequence analysis of samples

After the sample was concentrated with 10 kD vertical membrane, 8 M guanidine hydrochloride was added to a final concentration of 6 M, 1 M dithiothreitol was added to a final concentration of 20 mM at 56°C for 30 minutes; then, 1 M iodoacetamide was added to a final concentration of 50 mM, and the reaction was carried out in darkness for 30 minutes; and then, it was diluted with a 25 mM ammonium bicarbonate solution to 6 times of the initial volume, the enzyme was added, the enzymatic digestion was carried out overnight at 37°C, the salt was removed by a C18 mini-column, concentrated in vacuum, re-solubilised, and then loaded on the machine. Sequence alignment was performed using the data analysis software vanquish.

### Results:

The specimens tested in this experiment were sera from 14 patients with Alzheimer's disease, 44 patients with vascular dementia, 31 controls of the same age, and 100 persons who are medically healthy, and the basic information is shown in Table 1.

**Table 1. basic information of patients**

| | (Alzheimer's disease) AD | (vascular dementia) VD | (controls of the same age) Control | (persons who are medically healthy) Health |
|---|---|---|---|---|
| n | 14 | 44 | 31 | 100 |
| Sex (female) | 9 | 21 | 18 | 34 |
| Age (years) | 81(70-91) | 75(59-90) | 66(51-86) | 35(19-67) |
| <49(%) | 0(0) | 0(0) | 0(0) | 89(89) |
| 50-59(%) | 0(0) | 1(2.3) | 8(25.8) | 8(8) |
| 60-69(%) | 0(0) | 9(20.4) | 12(38.7) | 3(3) |
| 70-79(%) | 5(35.8) | 15(34.1) | 9(29) | 0(0) |
| 80-89(%) | 8(57.1) | 16(36.4) | 2(6.5) | 0(0) |
| 90-100(%) | 1(7.1) | 3(6.8) | 0(0) | 0(0) |
| MMSE | 8 | 11 | 30 | |
| CRP | 11.86 | 41.77 | 1.62 | |
| WBC | 6.79 | 6.95 | 5.72 | |

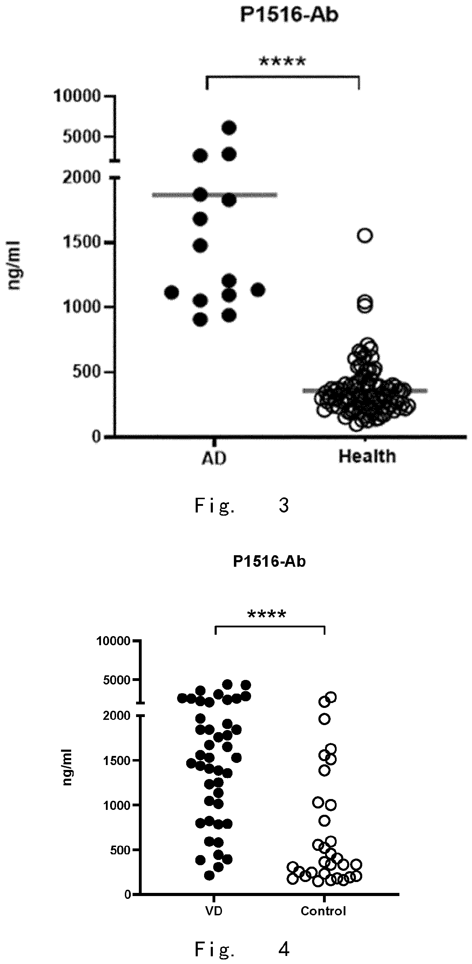

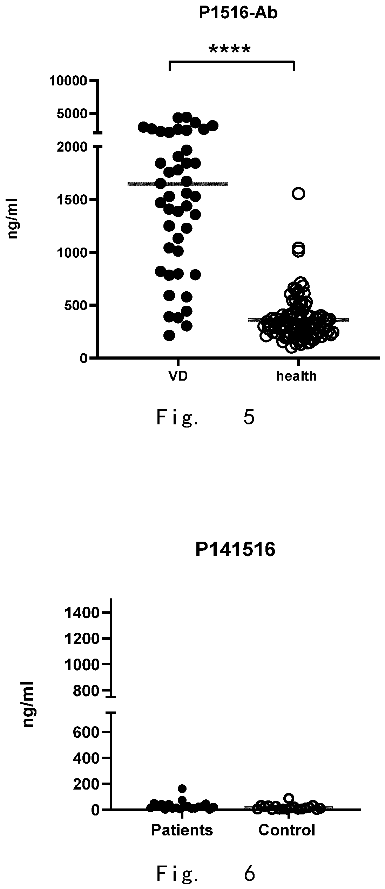

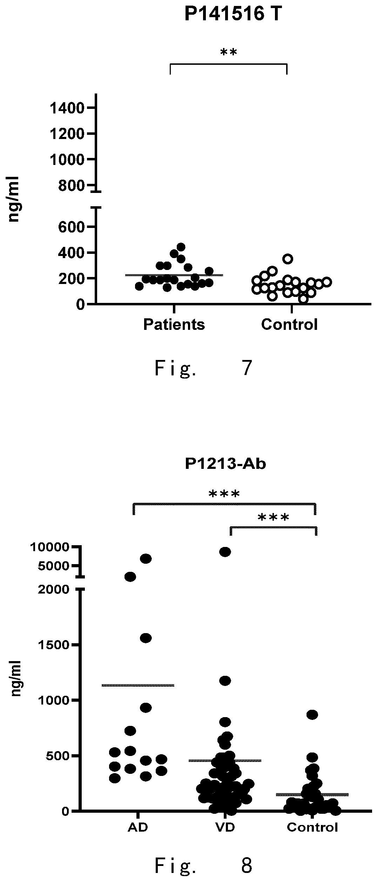

TNF was coated on a plate, serum samples were assayed and the concentration of corresponding antibodies was found to be significantly higher in Alzheimer's disease patients than that in the control group of the same age without cognitive impairment and the healthy control group (P < 0.01, Fig. 1). P1516 polypeptide was coated on a plate and serum samples were assayed, which showed that the concentration of the corresponding antibodies in the sera of patients with Alzheimer's disease was significantly higher than that of control group of the same age without cognitive impairment (P < 0.001, Fig. 2), and even more significantly higher than that of control group of healthy individuals (P < 0.0001, Fig. 3). The concentration of the corresponding antibody against P1516 was significantly higher in the serum from patients with vascular dementia than that in the control group of the same age without cognitive impairment and in the control group of healthy persons (P < 0.0001, Figs. 4, 5). In addition, antibodies were detected significantly higher than that in the control group using P141516T, P1213, and P1920 polypeptides (Figures 7-11). In contrast, using other peptides derived from TNF, such as P141516, antibodies were not detected significantly higher than that in the control group (Figure 6). Therefore, P1516, P141516T, P1213, and P1920 can be used as coating polypeptides for detecting or assisting in the diagnosis of Alzheimer's disease.

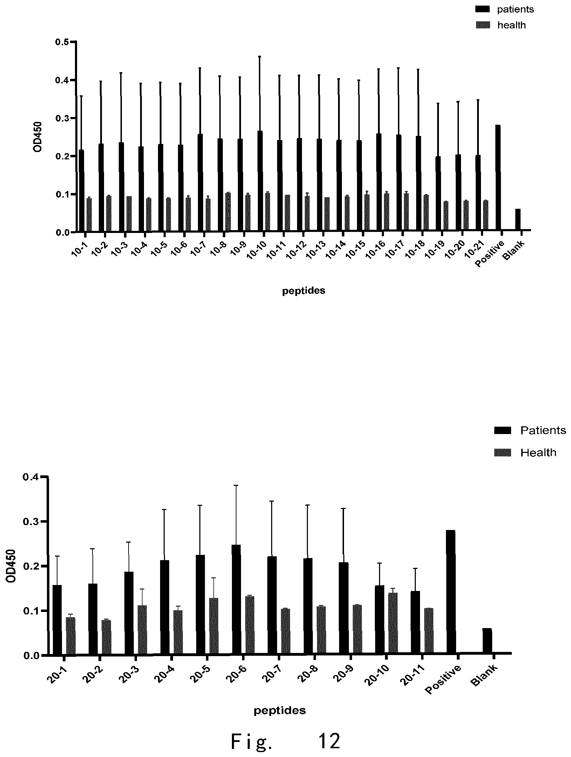

In contrast to P1516 of 30 amino acids, the present inventors designed a series of shorter polypeptides of 10 or 20 amino acid covering different parts of the sequence of P1516, i.e., 10-1 to 10-21, and 20-1 to 20-11. Samples with high concentrations of antibodies to serum P1516 were tested separately, and it was found that the corresponding antibodies in the serum could be efficiently detected using all of these polypeptides, whereas the results in healthy human blood samples were all negative (Fig. 12).

Comparing the use of the polypeptides of the present invention alone or in combination to detect relevant antibodies in sera of Alzheimer's disease (AD), vascular dementia (VD) patient, and control group of the same age, and control group of healthy individuals (Health), it was found that using the polypeptides of the present invention in combination exhibits a higher sensitivity for detecting the relevant autoantibodies (FIG. 15).

An immunoprecipitation product was obtained by co-incubating the serum of a patient with a high concentration of antibodies against TNF polypeptides with protein G, adsorbing the antibodies onto protein G gel beads, and then co-incubating with the serum of a patient with a high concentration of TNF-α. The product was dissociated from the protein G gel beads with glycine at pH 3.0, and the molecular weight of the co-precipitated product was analyzed by mass spectrometry, which revealed that the molecular weights of the major free small molecules in the product were identical to those of the P1516 control polypeptide; and the co-precipitated product was subjected to trypsin digestion, and peptide spectroscopic analysis revealed that most of the detected polypeptide sequences were concentrated in the regions ofP1213, P1516 and P1920 (Figure 13), indicating that there are polypeptides of comparable size and sequence similarity to P1213, P1516 and P1920 in the serum of dementia.

In order to identify the effects of these polypeptides on the nervous system, human neural microglioma HMC3 cells were studied in the present invention. HMC3 cells (1 × 10⁴/well) were co-cultured with LPS (1 ug/ml), IL-4 (20 ng/ml), TNF (20 ng/ml), a series of TNF derived polypeptides (50 uM/ml, P78-P2122) for 24h and then cck8 reagent was added to test cell survival. It was found that polypeptides such as P1516 and P1920 extremely significantly reduced the number of HMC3 viable cells compared to the control (Fig. 14), indicating that these polypeptides have killing and growth inhibiting effects on neural microglia.

All documents mentioned in the present invention are cited as references in the present application, as if each document was cited individually as a reference. It is further to be understood that after reading the foregoing teachings of the present invention, a skilled person may make various alterations or modifications to the present invention, and these equivalent forms will fall within the scope of the claims appended to the present application.

## Claims

1. Use of a TNF-derived polypeptide, wherein said TNF-derived polypeptide is used: (1) as a marker for the diagnosis or detection of dementia; (2) in the preparation of a reagent or kit for the diagnosis or detection of dementia; or (3) in the preparation of a medicament for treating dementia.

2. The use of claim 1, wherein said TNF-derived polypeptide is a polypeptide selected from one or more of the following:
1) a polypeptide having an amino acid sequence shown in any one of SEQ ID NO: 2-26; or,
2) a polypeptide having a sequence similarity of at least 80% or higher, 85% or higher, 90% or higher, 95% or higher, 96% or higher, 97% or higher, 98% or higher, 99% or higher, to a polypeptide having an amino acid sequence shown in any one of SEQ ID NO: 2-26.

3. The use of claim 1, wherein said dementia is Alzheimer's disease or vascular dementia.

4. A kit for diagnosing or detecting dementia, wherein said kit comprises a detection reagent and a container holding said detection reagent,
said detection reagent detects the presence of a TNF-derived polypeptide or an antibody capable of binding to said TNF-derived polypeptide in the serum of a subject to be tested, and said TNF-derived polypeptide is:
1) a polypeptide having an amino acid sequence shown in any one of SEQ ID NO: 2-26; or
2) a polypeptide having a sequence similarity of at least 80% or higher, 85% or higher, 90% or higher, 95% or higher, 96% or higher, 97% or higher, 98% or higher, 99% or higher, to a polypeptide having an amino acid sequence shown in any one of SEQ ID NO: 2-26.

5. A TNF-derived polypeptide, wherein said TNF-derived polypeptide is
1) a polypeptide having an amino acid sequence shown in any one of SEQ ID NO: 2-26; or
2) a polypeptide having a sequence similarity of at least 80% or higher, 85% or higher, 90% or higher, 95% or higher, 96% or higher, 97% or higher, 98% or higher, 99% or higher, to a polypeptide having an amino acid sequence shown in any one of SEQ ID NO: 2-26.

6. A combination of two or more TNF-derived polypeptides, wherein said TNF-derived polypeptide is:
1) a polypeptide having an amino acid sequence shown in any one of SEQ ID NO: 2-26; or
2) a polypeptide having a sequence similarity of at least 80% or higher, 85% or higher, 90% or higher, 95% or higher, 96% or higher, 97% or higher, 98% or higher, 99% or higher, to a polypeptide having an amino acid sequence shown in any one of SEQ ID NO: 2-26.

7. A binding agent or antagonist of TNF-derived polypeptide, wherein said TNF-derived polypeptide is:
1) a polypeptide having an amino acid sequence shown in any one of SEQ ID NO: 2-26; or
2) a polypeptide having a sequence similarity of at least 80% or higher, 85% or higher, 90% or higher, 95% or higher, 96% or higher, 97% or higher, 98% or higher, 99% or higher, to a polypeptide having an amino acid sequence shown in any one of SEQ ID NO: 2-26.

8. Use of a binding agent or antagonist of a TNF-derived polypeptide for the preparation of a medicament for treating dementia, wherein said TNF-derived polypeptide is:
1) a polypeptide having an amino acid sequence shown in any one of SEQ ID NO: 2-26; or
2) a polypeptide having a sequence similarity of at least 80% or higher, 85% or higher, 90% or higher, 95% or higher, 96% or higher, 97% or higher, 98% or higher, 99% or higher, to a polypeptide having an amino acid sequence shown in any one of SEQ ID NO: 2-26.

9. A pharmaceutical composition comprising a binding agent or antagonist of a TNF-derived polypeptide and optionally a pharmaceutically acceptable excipient, wherein said TNF-derived polypeptide is:
1) a polypeptide having an amino acid sequence shown in any one of SEQ ID NO: 2-26; or
2) a polypeptide having a sequence similarity of at least 80% or higher, 85% or higher, 90% or higher, 95% or higher, 96% or higher, 97% or higher, 98% or higher, 99% or higher, to a polypeptide having an amino acid sequence shown in any one of SEQ ID NO: 2-26.

10. A method for detecting or diagnosing dementia, said method comprising:
1) detecting the presence of a TNF-derived polypeptide or an antibody capable of binding to said TNF-derived polypeptide in the serum of a subject to be tested, wherein said TNF-derived polypeptide is a polypeptide having an amino acid sequence shown in any one of SEQ ID NO: 2-26, or a polypeptide having a sequence similarity of at least 80% or higher, 85% or higher, 90% or higher, 95% or higher, 96% or higher, 97% or higher, 98% or higher, 99% or higher, to a polypeptide having an amino acid sequence shown in any one of SEQ ID NO: 2-26;
2) determining that the subject to be tested has dementia, if said TNF-derived polypeptide or an antibody capable of binding to said TNF-derived polypeptide is present in the serum of the subject to be tested.

11. A prognostic method for dementia, said method comprising:
1) detecting the presence of a TNF-derived polypeptide or an antibody capable of binding to said TNF-derived polypeptide in the serum of a subject to be tested, wherein said TNF-derived polypeptide is a polypeptide having an amino acid sequence shown in any one of SEQ ID NO: 2-26, or a polypeptide having a sequence similarity of at least 80% or higher, 85% or higher, 90% or higher, 95% or higher, 96% or higher, 97% or higher, 98% or higher, 99% or higher, to a polypeptide having an amino acid sequence shown in any one of SEQ ID NO: 2-26;
2) determining that the subject to be tested having a poor prognosis for dementia, if said TNF-derived peptide or an antibody capable of binding to said TNF-derived peptide is present in the serum of a subject to be tested.
